# EUROPEAN PATENT SPECIFICATION

(11) **EP 4 009 904 B1**
(45) Date of publication and mention of the grant of the patent: **13.09.2023**
(21) Application number: 20765373.4
(22) Date of filing: 28.07.2020
(51) Int. Cl.: A61F 2/12, A61F 2/00

(54) **CONTAINER DEVICE FOR BREAST PROSTHESIS FOR RECONSTRUCTIVE BREAST SURGERY**
BEHÄLTERVORRICHTUNG FÜR BRUSTPROTHESE FÜR DIE REKONSTRUKTIVE BRUSTCHIRURGIE
DISPOSITIF DE CONTENANT POUR PROTHÈSE MAMMAIRE POUR CHIRURGIE MAMMAIRE RECONSTRUCTRICE

(30) Priority: 08.08.2019 IT 201900014193
(43) Date of publication of application: 15.06.2022
(73) Proprietor: DECO MED SRL, 30020 Marcon (VE) (IT)
(72) Inventor: BERTOLI, Giovanni, 30020 Marcon (VE) (IT)
(74) Representative: Braidotti, Andrea
(86) International application number: PCT/IT2020/050188
(87) International publication number: WO 2021/024284

(56) References cited:
- EP-A1- 3 056 167
- WO-A1-2014/041577
- IT-A1- TV20 120 148
- US-A- 2 842 775
- US-A- 3 293 663
- US-A1- 2017 189 208

## Description

The object of this patent application is the creation of a device to contain a breast prosthesis

### BRIEF DESCRIPTION OF RECONSTRUCTIVE BREAST SURGERY TECHNIQUE

Current surgery for reconstructing the breast following mastectomy is divided into two techniques that mainly differ in the number of phases necessary for the reconstructive process.

In the technique with two phases, the surgeon prepares a sub-muscular pocket by creating a space between the thoracic wall and the pectoralis major muscle, which is detached at the lower and medial node at the fourth intercostal space.

The procedure then proceeds laterally along the section of serratus anterior muscle.

A specific tissue expander is then inserted into said pocket.

Progressive insufflation follows over subsequent days until the desired expansion has been reached. The expander is then removed, but not before 4-6 months, through a second surgical operation removing the expander and inserting the final prosthesis.

The reconstructive technique in a single phase consists of positioning a breast prosthesis immediately after mastectomy into a sub-muscular pocket, created using the previously described technique.

The breast prosthesis is inserted into the sub-muscular cavity and covered at its lower node with a membrane composed of a bio-material.

The two techniques are used for small to medium size breasts.

The results of the two described techniques are similar from a physiological standpoint, because in both cases they require the dissection and resection of the pectoralis major muscle, requiring its dislocation and therefore substantial loss of movement.

With regard to the physiology of the pectoralis major muscle, it is confirmed that in the case of disconnection, the movements that may experience deficit are forward movement and bending of the arm, internal rotation, and adduction.

Furthermore, considering the movement of the shoulder and upper arm, the synergistic action of finely modulated various muscle groups should also be considered; even modest weakening can alter articulation in the shoulder with impact on normal activities in daily life.

The shoulder, both anatomically and functionally, is an extremely complex mechanism with very extensive movement capacity.

This requires implementing synergistic activation, adjustment and balance in the various muscle components in relation to control of multiple movement parameters (direction, distance, strength, resistance) to produce a qualitatively "normal", precise, smooth movement, that adapts to the required movement task at hand.

The movements that may be impacted by deficit, in the case of partial injury to the pectoralis major, are forward movement and bending of the arm, internal rotation and adduction, in lesser and greater degrees.

As noted, capsular fibrosis that forms around a breast prosthesis in silicone, both fibrotic and contractile, *Beker* 3 / 4, generates pain and imperfections on the patient's skin, but according to French academics of ASNM, it is also the source of agglomeration of T1 lymphocytes, which in rare cases can favor the occurrence of a new tumor: Anaplastic large-cell lymphoma (ALCL).

The solution to the two problems seems to be rapid surgical removal of the capsule and possible replacement of the silicone breast prosthesis.

Some studies (Cheng et al) have demonstrated that covering the silicone prosthesis with a biological material (ECM) effectively hides the presence of the synthetic material, silicone, from the body, when the covering is bio-compatible, inert and does not send signals that are recognized by the organism as foreign matter, to be encapsulated inside of a capsule that can then become fibrotic. The biological matrix (ECM) is bio-compatible, bio-active, non-inert, and is recognized by the organism as if it is its own tissue to be regenerated, and is in fact incorporated as self-tissue.

The reason for this is because the membrane in a biological matrix (ECM) is composed of collagen and protein, which among mammals is highly conserved, and therefore, the human body recognizes it as its own.

Subsequently, a silicone - human tissue interfacing favors the formation of an isolating capsule that can become fibrotic, since the body does not recognize the synthetic material and enacts a response to foreign matter.

On the contrary, interfacing between a collagen-based biological matrix and human tissue incorporates the first into the formation of a thin, well-veined peri-prosthetic connective tissue. The need to completely cover a breast implant with active biological material (ECM) is acknowledged by the scientific community today.

The purpose is to prevent contact between the breast implant silicone and the surrounding tissue, limiting the risk of the formation of a contractile capsule with resulting possible agglomeration of T-lymphocytes.

Natural polymers technology uses hydration and dehydration processes and mechanical pressing to shape flat sheets of extracellular matrix according to the desired shape, also solid forms derived from a sphere, when dehydrated at the end of the process, giving them a specific shape and memory of form.

In fact, it should be noted that extracellular matrix (ECM is composed of dense parallel layers of collagen fibers, while the layers of elastin tissue are destroyed by the deantigenation process. Expelling water and air from the extracellular matrix is performed by compressing said collagen fibers, which once pressed to specific values and dried, in the absence of functional elastin, maintain their industrial form.

The transformation of the biological matrix (ECM) into self-tissue (incorporation) occurs in physical conditions characterized by intimate contact between the biological matrix (ECM) and the vascularized strip of tissue. The significance of intimate contact resides in the fact that the more interfacing points between the matrix and the vascularized tissue there are, the greater the possibility of transforming the matrix into self-tissue. The conformity of the matrix to the implant site plays an important role in ensuring optimum interfacing to obtain maximum contact.

If the site of the implant is cylindrical, to obtain maximum contact between the matrix and the site it is necessary to use a cylindrical shaped matrix.

If the site of the implant is a flat surface, a flat sheet is necessary.

According to the technique described in the patent application EP2903563, a technique consists of the positioning of a silicone prosthesis wrapped with a flat shaped sheet of collagen biological matrix over the pectoralis major muscle.

Therefore the description in the document EP2903563 is a solution for the various problems that derive from detachment of the pectoralis major muscle from its natural position.

Nonetheless, said device requires a tailor-made preparation of a collagen matrix fabric around the silicone prosthesis. This action must be carried out with clamp, scissors, and suture thread to render the fabric in the right form for the selected prosthesis.

During the preparation time, which varies from 30 to 45 minutes, the manipulation of the collagen matrix comports higher risk of infection.

Furthermore, the preparation described above is not always suitable in consideration of the varied skills of the surgeon in "cut and sew" operations.

The ITTV20120148 patent application describes a breast prosthesis that consists of a fixed structure composed of three elements: a central nucleus in a filler material, an intermediate shell that sets a desired and controlled form for the central nucleus, and an external shell in bio-material with a flat posterior surface fitted with appendages that can be sutured to the pectoralis major muscle.

The invention described in the document ITTV20120148 has a series of appendages designed to favor the suturing process to the muscle, an operation that requires the application of sixteen - eighteen stitches.

The patent application US2842775 describes a prosthesis made up of a first and second element, where said second element is physically distinct from the first and is inserted into the same, where the final single unit made from the "first element and second element" favor preventing the complete absorption of fluids, as well as preventing rigidity in the prosthesis.

According to the invention described in the document US2842775, the second element has a channel with a cannula that fills the element with organic fluids.

In particular, the document US2842775 describes mixed surgical prostheses for subcutaneous insertion in the patient to replace the removed organs of the body, defined by the external walls, consisting entirely of resistant plastic surgical spongy material, which has a entry point in the exterior part that extends along the entire interior part in which the living tissues and fluids can penetrate, since this channel is an access into the wall of the device, with a waterproof sac of fluid inserted into said hollow device through the channel, a filling tube joined to the sac and accessible through the channel, said tube suitable for holding the fluid and yielding inside of the sac.

The item described in the patent application EP3056167 describes a medical support device that essentially is composed of a back wall, a front curved wall and a pocket made between the front and back parts to house a breast prosthesis characterized by the fact that the area of the back wall is substantially wider than the projected area of the front part.

According to the description and claims in document EP3056167 the device has a hemispherical form and the surface of the back wall is greater in a variable percentage between 50% - 100% than the surface of the front wall. The item described in document D3 EP3056167, as conceived and explained, does not permit complete closure action of the wall on the hemispherical portion containing the prosthesis.

The item described in the patent application US3293663A describes a breast prosthesis constituted by two physically distinct elements, of which one including a flexible container with a breast shape, a soft gel, and a structure that has a wave-like surface attached to a part of the container in a way that the tissue can grow through the wave-like structure and anchor the prosthesis to the thoracic wall.

According to the description and claims for the item that is the object of patent application US3293663A, a breast prosthesis filled with a soft gel, preferably silicone-based, with a front wall and a back wall, with said back wall with smaller areas in porous material, creating waves to allow growth of the patient's tissue to anchor to the thoracic wall.

The back wall is in a porous material that allows the tissue to penetrate, but the material is not absorbed by the body.

According to the description in document US3293663A, filling with gel takes place once the covering has been positioned.

The document US2017189208 describes an external breast prosthesis comprised of a shell in flexible material with the shape of a breast, said shell forming an internal cavity protected by a cover, which is a face of the breast prosthesis, called the external face, which is suitable for pressing onto the chest of the person, said cavity containing elements for adjustment of the weight of the prosthesis, where the element that creates weight is composed of ballast elements, distributed along the sagittal plane of the prosthesis, substantially orthogonal to the plane of the cover.

According to the description in document US2017189208, the device is designed to be positioned outside of the body to serve as a temporary simulation of a breast, with discs in different weights and shapes inserted inside of it.

The device described in document US2017189208 is therefore not suitable for being permanently and irreversibly inserted into the human body.

The main function of the object of this invention is therefore to resolve the technical and medical issues mentioned above, eliminating the aforesaid problems in the technique and providing an invention that permits reconstructing the breast after mastectomy without compromising the function of the pectoral muscle, but reducing the operating and handling times with the sterile matrix to adapt it to the chosen size of the prosthesis, with drastic reduction in the risk of infection. Another purpose, no less important, is to define an invention that limits capsular contraction in the prosthesis.

Another purpose of the invention in this patent application is to create a device that allows obtaining optimum interface to achieve maximum contact between the matrix and the site.

The object of this invention is therefore to be a device that covers a breast prosthesis with the characteristics defined in the following claims, which constitute an integral part of this description. The object of this patent application consists of a medical device for breast reconstruction, composed of a container body, which is composed of a half shell and a flat appendage as according to claim 1.

In a preferential but not exclusive form, the half shell (2) has a hollow spherical dome shape, joined continuously to the appendage (3) constituted by a flat sheet with a preferably but not exclusively round or oval shape defining the base.

Additional characteristics and advantages of the device described as the invention are evident in the following detailed description, made with reference to the attached drawings, referring to the commonly adopted form for implementation, but not imperative and not restrictive with regard to the invention, in which the product demonstrates a configuration with half shell and flat base.

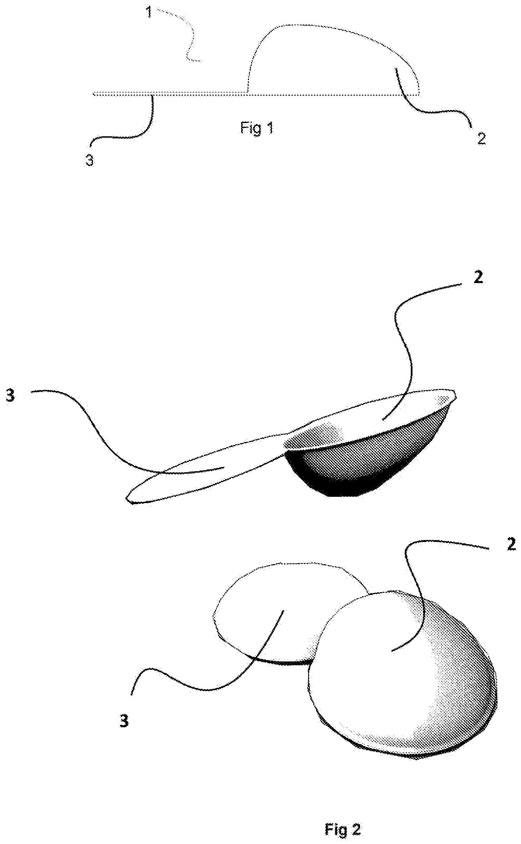

Fig. 1 demonstrates a side view of the container device in an open configuration;
fig. 2 demonstrates an upper and lower view of the container device in an open configuration;
fig. 3 demonstrates the container device in a close configuration containing a breast prosthesis;
fig. 4 demonstrates the container device and positioning of the breast prosthesis.

With reference to the attached Figures, the container device that is the object of this patent application in an open configuration (1) is composed of a half shell (2) in a substantially complementary form to the external surface of a breast prosthesis (4) and a flat appendage (3) associated without a continuous solution to a portion of the base of the half shell (2).

According to the invention that is the object of this patent application, the external surface of the half shell (2) and the external surface of the appendage (3) together form the container body in the closed configuration, said container body (1.1) with a flat base in correspondence to the coupling plane of the appendage (3) to the half shell (2).

According to the invention in the object of this patent application, the surface of the appendage (3) is greater than or equal to the area of the base of the half shell (2).

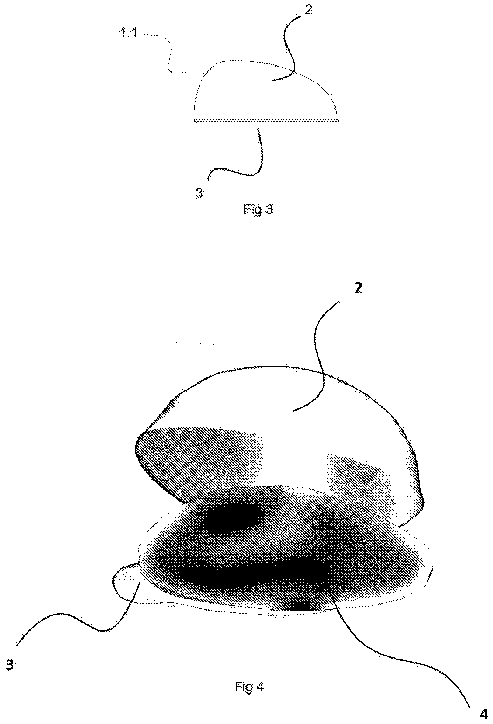

As demonstrated in Fig. 3 and 4, once the breast prosthesis is inserted (4), the perimeter of the appendage (3) is sutured to the perimeter of the base of the half shell (2) to obtain the container device in the closed configuration (1.1).

Subsequently, to overlap the appendage (3) on the half shell (2) to obtain a container (1.1) with a flat base to contain or containing or integrating a breast prosthesis (4), where said container (1.1) can be inserted between the skin and the pectoralis major muscle and sutured to the latter.

In a preferential but not exclusive form, the half shell (2) has a hollow spherical dome shape, joined continuously to the appendage (3) defining the base of the same dome.

In a preferential but not exclusive form, the surface of the appendage (3) is circular.

In a preferential but not exclusive form, the surface of the appendage (3) is oval.

Independently of the preferential but not exclusive forms, once the breast prosthesis (4) is inserted into the half shell (2), the border that constitutes the perimeter of the appendage (3) is sutured to the lower border of the half shell (2), constituting the perimeter of the base of the half shell (2) itself, therefore obtaining a container device (1.1) that completely encloses said breast prosthesis inside of it (4), which is then sutured to the pectoralis major muscle.

The flat base of the container device (1.1) constitutes a platform for attaching the half shell (2) to the pectoralis major muscle, and for guaranteeing stability over time of the anatomical form, also when subjected to the force of gravity.

In practice, it was observed that the invention reached the aforesaid objectives and purpose, having obtained a medical device that can be used in applications and not compromising the functionality of the pectoralis major muscle, preventing any muscular injury between the median part and the clavicle, sternum, cartilage of the first six / seven ribs, external oblique muscle (median) and crest below the greater tuberosity of the humerus (lateral).

The aforesaid device allows maintaining movement of the pectoralis major muscle, avoiding the need for rehabilitation therapy and eliminating costs associated with this disability.

The device also results in a drastic reduction in post-operative pain, eliminating the risk of muscle contraction, the muscle allows maintaining the stock in the case of a revision, and requires less surgery time and less complexity in the operation, given that no prosthetic materials are dislocated, being dynamic and static, and with less bleeding.

Said device drastically reduces the risk of capsular contraction caused by contact between the silicone and the surrounding tissues, and the risk of requiring another operation to remove the capsule due to the occurrence of a new type of tumor: ALCL.

The result is a superior aesthetic effect, both in the short and long-term.

Another advantage of this invention, which also exceeds patent EP2903563, is the drastic reduction in operating times. The device that is the object of this patent application, consists of a flexible container composed of a concave cup inside of which the breast prosthesis is housed and then subsequently closed to create a single body that does not require placement through the suturing of various septa. The decrease in maneuvers also decreases the risk of infection. In the place of the sixteen / eighteen suture stitches necessary for packaging a device with several septa, only three or four stitches are necessary for completing the union between the base to the dome.

The operating times are also reduced by 30 minutes, influencing the overall reduction of 25% in the time necessary for the surgery, with certain positive impacts in the reduction of post-operative complications.

The appendage (3) is united continuously to the half shell (2) so that three stitches are sufficient for inserting the flexible container holding the silicone prosthesis.

The complete enclosure, also in the back, of the prosthesis through the invention constituted of biological matrix (ECM) prevents contact between the silicone and the surrounding tissues, with the certain advantage of not activating the anti-inflammatory response that is typically enacted by foreign matter when tissue and synthetic material make contact.

In short, the device is beneficial for the patient from these points of view, given that psychologically speaking the patient benefits from the rapid operation in only one sitting, with a simplified technique, which eliminates the need to maneuver muscles, can be carried out in a much quicker time span, with less bleeding and a reduction in loss of blood and plasma, with a lowered risk both during and following the operation, also from a physiological standpoint, the patient will not be subjected to any loss of movement caused by the resection / disconnection of the pectoral muscles and will not be subjected to the same level and extension of clinical pain as in the previous surgical procedure, and the recovery time will be significantly reduced, improving the professional life of the patient with a more rapid return to the workplace.

It will be possible for the patient to carry out the same physical activities as without said device, maintaining a high quality of life in their daily activities.

Anatomically speaking, the pectoralis major muscle remains intact and its structure is maintained complete, and it will not be involved in the procedure and left for any subsequent operations that could involve the muscle fascia.

The materials used and the dimensions that constitute the single components of the invention may be selected based on specific requirements.

The various means for implementing different functions certainly must not co-exist only in the illustrated form, but can also take on many different forms not illustrated here, within the scope of the appended claims.

## Claims

1. Medical device for breast reconstruction for containing a breast prosthesis (4), said device being designed for placement between the skin and the pectoralis major muscle, able to be sutured to the same muscle, constituted by a container body (1;1.1) which is constituted by a half shell (2) having a hollow dome shape, the internal surface of said half shell (2) having a form substantially complementary to the external surface of the breast prosthesis (4) to be contained, and by a flat appendage (S), said device having an open configuration and a closed configuration wherein the perimeter of the appendage (3) is sutured to the perimeter of the base of the half shell (2) to obtain a container device for the breast prosthesis (4) that completely encloses said breast prosthesis inside of it (4), **characterized in that** in said open configuration the half shell (2) is associated with the flat appendage (S), wherein the flat appendage (S) is joined continuously to a portion of the base of said half shell (2), and **in that** in said open configuration said flat appendage (S) is extending outwards in respect to said portion of the base of said half shell (2).

2. Medical device according to claim 1 **characterized in that** the half shell (2) and the flat appendage (3) together form the container body (1.1) with a flat base corresponding to the coupling surface of the appendage (3) to the half shell (2).

3. Device according to the previous claims **characterized in that** the surface of the appendage (3) is greater than or equal to the area of the base of the half shell (2).

4. Device according to any of the previous claims **characterized in that** the appendage (3) has a circular shape.

5. Device according to any of the previous claims 1,2, 3 **characterized in that** the appendage (3) has an oval shape.

6. Device according to the previous claims **characterized in that** it is made in bio-material.

7. Device according to the previous claims **characterized in that** in said open configuration said flat appendage (S), that extends outwards in respect to said portion of the base of said half shell (2), is coplanar to said portion of the base of said half shell (2).

## Patentansprüche

1. Medizinprodukt für die Brustrekonstruktion zur Aufnahme einer Brustprothese (4), besagtes Produkt ist für die Platzierung zwischen der Haut und dem Musculus pectoralis major entworfen und kann mit Suturstichen an diesen Muskel genäht werden; es setzt sich aus einem Behältergehäuse (1;1.1), das aus einer Halbschale (2) mit Hohlkuppelform besteht, wobei die Innenfläche der besagten Halbschale (2) eine Form aufweist, die im Wesentlichen ergänzend zur Außenfläche der Brustprothese (4) ist, die sie enthalten muss, und aus einem flachen Anhang (S) zusammen, wobei dieses Produkt eine offene und eine geschlossene Konfiguration aufweist, worin die Umfangslänge des Anhangs (3) mit Suturstichen an die Umfangslänge der Basis der Halbschale (2) genäht wird, um eine Behältervorrichtung für die Brustprothese (4) zu erhalten, die besagte Brustprothese vollständig in ihrem inneren (4) einschließt und **dadurch gekennzeichnet ist, dass** die Halbschale (2) in der besagten offenen Konfiguration an den flachen Anhang (3) angeschlossen ist, worin der flache Anhang (S) fortlaufend mit einem Abschnitt der Basis besagter Halbschale (2) verbunden ist, sowie dadurch, dass sich der besagte flache Anhang (S) in der besagten offenen Konfiguration im Verhältnis zum besagten Abschnitt der Basis besagter Halbschale (3) nach außen hin verlängert.

2. Medizinprodukt gemäß Anspruch 1, **gekennzeichnet dadurch, dass** die Halbschale (2) und der flache Anhang (3) gemeinsam die Behältervorrichtung (1.1) bilden, mit einer flachen Basis, die der Verbindungsfläche des Anhangs (3) mit der Halbschale (2) entspricht.

3. Produkt gemäß den vorherigen Ansprüchen, **gekennzeichnet dadurch, dass** die Oberfläche des Anhangs (3) größer oder gleich groß wie die Fläche der Basis der Halbschale (2) ist.

4. Produkt gemäß allen vorherigen Ansprüchen, **gekennzeichnet dadurch, dass** der Anhang (3) kreisförmig ist.

5. Produkt gemäß allen vorherigen Ansprüchen, 1, 2, 3, **gekennzeichnet dadurch, dass** der Anhang (3) eine ovale Form hat.

6. Produkt gemäß den vorherigen Ansprüchen, **gekennzeichnet dadurch, dass** es aus Biomaterial gemacht ist.

7. Produkt gemäß den vorherigen Ansprüchen, **gekennzeichnet dadurch, dass** besagter flacher Anhang (S), der sich im Verhältnis zum besagten Abschnitt der Basis besagter Halbschale (2) nach außen hin verlängert. in der besagten offenen Konfiguration komplanar zum besagten Abschnitt der Basis besagter Halbschale (2) ist.

## Revendications

1. Dispositif médical de reconstruction mammaire destiné à recevoir une prothèse mammaire (4), ledit dispositif étant conçu pour être placé entre la peau et le muscle grand pectoral et pouvant être suturé à ce muscle ; il se compose d'un corps conteneur (1;1.1) qui est constitué d'une demi-coque (2) en forme de dôme creux, la surface interne de ladite demi-coque (2) présentant une forme sensiblement complémentaire de la surface externe de la prothèse mammaire (4) qu'elle doit contenir, et d'un appendice plat (S), ayant une configuration ouverte et une configuration fermée, dans laquelle la longueur périphérique de l'appendice (3) est suturé au périmètre de la base de la demi-coque (2) afin d'obtenir un dispositif de contention pour la prothèse mammaire (4), la dite prothèse mammaire est complètement enfermée à l'intérieur (4) et a la caractéristique que la demi-coque (2) est reliée à l'appendice plat (S) dans ladite configuration ouverte, dans laquelle l'appendice plat (S) est relié de manière continue à une partie de la base de ladite demi-coque (2), et que ledit appendice plat (S) s'étend vers l'extérieur par rapport à ladite partie de la base de ladite demi-coque (S) dans ladite configuration ouverte.

2. Dispositif médical selon la revendication 1, **caractérisé en ce que** la demi-coque (2) et l'appendice plat (3) forment ensemble le dispositif conteneur (1.1), avec une base plate qui correspond à la surface de liaison de l'appendice (3) avec la demi-coque (2).

3. Dispositif selon les revendications précédentes, **caractérisé en ce que** la surface de l'appendice (3) est supérieure ou égale à la surface de la base de la demi-coque (2).

4. Dispositif selon toutes les revendications précédentes, **caractérisé en ce que** l'appendice (3) est circulaire.

5. Dispositif selon toutes les revendications précédentes, 1, 2, 3, **caractérisé en ce que** l'appendice (3) a une forme ovale.

6. Dispositif selon les revendications précédentes, **caractérisé en ce qu'**il est fait de biomatériau.

7. Dispositif selon les revendications précédentes, **caractérisé en ce que** ledit appendice plat (S), qui se prolonge vers l'extérieur par rapport à ladite portion de la base de ladite demi-coque (2), est coplanaire avec ladite portion de la base de ladite demi-coque (2) dans ladite configuration ouverte.
